# EUROPÄISCHE PATENTSCHRIFT

(11) **EP 0 559 702 B1**
(45) Veröffentlichungstag und Bekanntmachung des Hinweises auf die Patenterteilung: **28.12.1994**
(21) Anmeldenummer: 91920517.9
(22) Anmeldetag: 26.11.1991
(51) Int. Cl.: A61K 9/22, A61K 9/52, A61K 31/505

(54) **RETARDZUBEREITUNG FÜR URAPIDIL**
RETARDED-ACTION URAPIDIL FORMULATION
RETARDATEUR D'URAPIDIL

(30) Priorität: 27.11.1990 CH 3763/90
(43) Veröffentlichungstag der Anmeldung: 15.09.1993
(73) Patentinhaber: BYK GULDEN LOMBERG CHEMISCHE FABRIK GMBH, D-78403 Konstanz (DE)
(72) Erfinder: BENEDIKT, Gerald, D-7750 Konstanz 19 (DE)
(86) Internationale Anmeldenummer: EP9102227
(87) Internationale Veröffentlichungsnummer: WO9209273

(56) Entgegenhaltungen:
- FR-A- 2 237 620

## Beschreibung

### Technisches Gebiet

Die Erfindung bezieht sich auf eine Retardzubereitung für Urapidil.

### Stand der Technik

Der Wirkstoff 6-({3-[4-(o-Methoxyphenyl)-1-piperazinyl]propyl}amino)-1,3-dimethyluracil (INN: Urapidil) ist ein bekannter Wirkstoff zur Therapie von Bluthochdruck und auch von benigner Prostatahyperplasie.

Aufgrund seiner interindividuell stark differierenden Pharmakokinetik und seiner relativ kurzen Eliminationshalbwertszeit von 2,7 Stunden bereitet es außergewöhnliche galenische Schwierigkeiten, eine geeignete Zubereitung für die chronisch orale Therapie zu schaffen, die stets wirksame Blutspiegel gewährleistet, aber Blutspiegelspitzen, die zu unerwünschten Nebenwirkungen, wie z.B. Orthostase führen, vermeidet. Besonders wünschenswert ist eine Arzneiform, die es erlaubt, die gesamte Tagesdosis auf einmal oder maximal zweimal einzunehmen. Dies ist insbesondere bei der Hypertonie wichtig, da diese Krankheit dem Patienten oftmals keine Beschwerden bereitet, die ihn an die Einnahme des Medikaments erinnern. Bei der Bluthochdruckbehandlung ist deshalb eine die Patienten-Compliance begünstigende Darreichungsform von besonderer Bedeutung.

Aus der DE-C3-2336218 ist eine orale Depotarzneiform bekannt, bei der der Arzneistoff in Form sphäroider Teilchen vorliegt, die mit einer Dialysemembran überzogen sind, die aus einem unlöslichen Celluloseether und einer freie Carboxylgruppen aufweisenden organischen Verbindung besteht. Nach dieser Lehre ist es möglich, Urapidil enthaltende Retardzubereitungen herzustellen, die den Wirkstoff in vitro weitgehend pH-unabhängig über einen langen Zeitraum freigeben. Versuche der Anmelderin zeigen, daß es nach der Lehre der genannten Schrift zwar möglich ist, Urapidil-Retardformulierungen zu bereiten, die in vitro ein Freisetzungsverhalten zeigen, die sie für eine täglich einmalige Verabreichung geeignet erscheinen lassen. Jedoch zeigen humankinetische Untersuchungen, daß die Bioverfügbarkeit von solchen stark retardierten Zubereitungen auf unannehmbar kleine Werte zurückgeht. So erreicht eine Zubereitung, die in vitro eine Freisetzung über 15 Stunden zeigt, in vivo eine nicht mehr vertretbare Bioverfügbarkeit von nur noch 34 %. Nach dieser Methode können daher nur Urapidil-Depotarzneiformen hergestellt werden, die mindestens zweimal täglich verabreicht werden müssen.

Auch Versuche der Anmelderin mit anderen üblichen Retardierungsverfahren unter Verwendung von Acrylharzen (Eudragit®) (Lehmann, Dreher, Pharm. Ind., 31 [1969] 319 - 322 und 409 - 412; Lehmann, Pharm. Ind., 3 [1971] 34 - 41) ergeben, daß es nicht möglich ist, Urapidil-Zubereitungsformen herzustellen, die bei täglich einmaliger oder zweimaliger Gabe in vivo eine annehmbare Bioverfügbarkeit aufweisen.

In US-A-4629619 und US-A-4629620 sind Verfahren zur Herstellung von mit porösen Membranen beschichteten Retardtabletten beschrieben. Nach den Versuchen der Anmelderin lassen sich nach diesen Verfahren Urapidil-Retardtabletten formulieren, die eine lineare Wirkstoffabgabe von bis zu 16 Stunden aufweisen. Die bei humankinetischen Untersuchungen ermittelte Bioverfügbarkeit liegt bei 68 % und damit noch im Grenzbereich des Tolerierbaren. Allerdings zeigt sich, daß diese Formulierungen einen dramatischen Abfall der Bioverfügbarkeit in Verbindung mit der Nahrungsaufnahme zeigen und somit in der Praxis nicht für nur einmal oder zweimal täglich einzunehmende Formulierungen einsetzbar sind.

Aus der EP-A-0275444 der Anmelderin ist eine Urapidil-Retardformulierung bekannt, bei der Urapidil zusammen mit aliphatischen Dicarbonsäuren in Pellets eingearbeitet wird, die mit einem magensaftresistenten Überzug versehen werden. Diese Formulierung ergab bei nüchternen Patienten und bei solchen mit einem leichten Standardfrühstück durchaus brauchbare Blutspiegel über einen Zeitraum, der es sogar erlauben würde, sie täglich einmal zu verabreichen. Aber auch hier zeigte sich, daß bei unkontrollierter normaler Nahrungsaufnahme eine nicht akzeptable Absenkung der Bioverfügbarkeit eintritt.

Aus der DE-C-2328409 ist ein nach dem Prinzip der osmotischen Pumpe arbeitende Formulierung bekannt, bei der ein beschränkt löslicher Wirkstoff zusammen mit einer osmotisch wirksamen Substanz von einer semipermeablen Hülle umgeben ist, die einen Durchlaß zum Austritt des Wirkstoffs aufweist.

Die Theorie der Wirkstofffreigabe solcher Formulierungen ist bekannt (F. Theeuwes, J. Pharm. Sci., 64 [1975] 1987 - 1991). So ergibt sich nach dieser Theorie z.B. für Urapidil-Hydrochlorid aufgrund seiner sehr guten Löslichkeit in Wasser (710 mg/ml bei 37°C), daß aus einer solchen Formulierung weniger als 30 % des Wirkstoffs linear, d.h. mit einer Geschwindigkeit nullter Ordnung abgegeben werden. Eine Nacharbeitung einer solchen Formulierung hat sogar ergeben, daß die Freisetzung in keinem Intervall mit nullter Ordnung erfolgt. In der genannten Druckschrift wird angegeben, daß für Wirkstoffe mit einer Löslichkeit von weniger als 1 Gew.-% neben anorganischen Salzen, Harnstoff, Inositol, Weinsäure, Rohrzucker, Raffinose, Glukose und α-d-Lactosemonohydrat als osmotisch wirksame Substanzen verwendet werden können.

### Beschreibung der Erfindung

Es wurde nun gefunden, daß es bei Verwendung von Zuckern und/oder Zuckeralkoholen mit einer Löslichkeit von weniger als 30 Gew.-% in Wasser von 37°C als Hilfsstoffe überraschenderweise möglich ist, auch leichtlösliche Salze von Urapidil in einer nach dem Prinzip der osmotischen Pumpe arbeitenden Darreichungsform bereitzustellen, die sich aufgrund ihrer linearen Abgabecharakteristik über einen genügend langen Zeitraum und die von einer Nahrungsaufnahme unbeeinflußte hohe Bioverfügbarkeit hervorragend für eine täglich einmalige oder zweimalige Verabreichung eignen. Es wurde außerdem gefunden, daß sich diese Retardzubereitung insbesondere für die Behandlung der benignen Prostatahyperplasie eignet.

Gegenstand der Erfindung ist daher eine Retardzubereitung für Urapidil nach dem Prinzip der osmotischen Pumpe, bei der sich der Wirkstoff zusammen mit Hilfsstoffen als Kern in einer mit einem Durchlaß versehenen semipermeablen Hülle befindet, dadurch gekennzeichnet, daß als Wirkstoff ein leichtlösliches Salz von Urapidil und als Hilfsstoffe Zucker und/oder Zuckeralkohole mit einer Löslichkeit von weniger als 30 Gew.-% in Wasser von 37°C enthalten sind.

Weitere Gegenstände der Erfindung ergeben sich aus den Patentansprüchen.

Vorzugsweise sind die Zucker und/oder Zuckeralkohole in einer Menge von mindestens 10 Gew.-% bezogen auf das Gewicht des Kerns zugegen.

Als Zucker und/oder Zuckeralkohol werden vorzugsweise Lactose und/oder Mannit verwendet.

Die Zusammensetzung der Retardzubereitung wird vorzugsweise so gewählt, daß mehr als 70 Gew.-% des Urapidilsalzes linear freigesetzt werden.

Unter einem leichtlöslichen Urapidilsalz im Sinne der vorliegenden Erfindung wird ein Salz des Urapidils verstanden, das in Wasser von 37°C eine Löslichkeit von mehr als 300 mg/ml aufweist. Bevorzugt wird Urapidilhydrochlorid.

Das leicht lösliche Urapidilsalz wird zusammen mit dem oder den Zuckern und/oder dem oder den Zuckeralkoholen in Gegenwart von üblichen Hilfsstoffen nach Standardverfahren zu Kernen verarbeitet. Als Hilfsstoffe kommen die üblichen Bindemittel, Farbstoffe, Gleitmittel und Dispergiermittel in Frage. Als Bindemittel kommen beispielsweise Polyethylenglykol, Gelatine, Agar, Carboxycellulose, Polyvinylalkohol und Polyvinylpyrrolidon in Betracht. Typische Gleitmittel umfassen Stearinsäure, Magnesiumstearat, Zinkstearat, Aluminiumstearat, halogenierte pflanzliche Öle und Talkum.

Für die Herstellung der semipermeablen Hülle werden Materialien verwendet, die für Wasser durchlässig und für gelöste Substanzen undurchlässig sind. Diese Materialien sind dem Fachmann bekannt. Er kann sie z.B. den zahlreichen Schutzrechten auf dem Gebiet der Zubereitungen nach dem Prinzip der osmotischen Pumpen entnehmen. Die semipermeable Hülle ist im wesentlichen inert und behält ihre physikalische und chemische Integrität während der Verabreichungsdauer aus der Dosierungform bei. Der Ausdruck "behält ihre physikalische und chemische Integrität bei" bedeutet, daß die semipermeable Hülle ihre Struktur beibehält und sich während der Lebensdauer bzw. Verabreichungszeit der Dosierungsform nicht verändert. Die semipermeable Hülle umfaßt vorzugsweise eine Substanz ausgewählt aus Celluloseestern, Celluloseethern und Celluloseesterethern. Bei einer besonders bevorzugten Ausführungsform enthält die semipermeable Hülle eine Substanz ausgewählt aus der Gruppe Celluloseacylat, Cellulosediacylat, Cellulosetriacylat, Celluloseacetat, Cellulosediacetat, Cellulosetriacetat und Ethylcellulose. Die die semipermeable Hülle bildenden polymeren Substanzen sind u.a. Celluloseacetat mit einem Substitutionsgrad von 1 und einem Acetylgehalt bis zu 21 %, Cellulosediacetat mit einem Substitutionsgrad von 1 bis 2 und einem Acetylgehalt von 21 bis 35 %, Cellulosetriacetat mit einem Substitutionsgrad von 2 bis 3 und einem Acetylgehalt von 35 bis 44 % und Ethylcellulose mit einem Ethoxygruppen-Substitutionsgrad von 1,5 bis 3, einem Ethoxygehalt von etwa 40 bis 50 % und einer Viskosität im Bereich von 7 bis 100 mPa x (7 bis 100 cp) oder darüber. Die Menge an Cellulosepolymer in der semipermeablen Hülle der Dosierungsform beträgt im allgemeinen 65 bis 100 Gew.-%.

Der Durchlaß in der semipermeablen Hülle wird mechanisch oder vorzugsweise mit Hilfe eines Laserstrahls gebohrt. Der Durchmesser des Durchlasses kann zwischen 0,1 und 0,5, vorzugsweise 0,2 bis 0,4 mm gewählt werden.

Die Abb. 1 zeigt die Wirkstoffreisetzung erfindungsgemäßer Retardzubereitungen. Die Abb. 2 und 3 stellen zum Vergleich Wirkstoffreisetzungen von nicht erfindungsmäßen Retardzubereitungen dar.

### Herstellungsbeispiele

**1.** 3850 g Urapidilhydrochlorid und 1925 g Milchzucker werden gemischt und in den Materialbehälter eines Wirbelschichtsprühgranulators TYP WSG 5 der Fa. Glatt gesiebt. Eine Lösung von 525 g Kollidon®25 (Poly-N-vinylpyrrolidon mit einem mittleren Molekulargewicht von ca. 25000 der BASF AG) in 3 l Wasser wird auf diese Mischung aufgesprüht und das entstandene Granulat wird bis zu einer relativen Feuchte von 20 - 50 % getrocknet. Dem getrockneten Granulat werden 105 g Magnesiumstearat zugemischt. Dieses Granulat wird zu Kernen à 273 mg (= 150 mg Urapidil) verpreßt. Die Kerne werden mit einem Filmüberzug von Celluloseacetat mit einem Acetylgehalt von 32 % überzogen bis die Schichtdicke 52 µm beträgt. In diese Celluloseacetatschicht wird ein 0,3 mm großes Loch gebohrt.
   Die Freisetzung wird nach der Paddle-Methode (USP XXI) durch Messung der Absorption von Urapidil bei 269 nm ermittelt.
   Die Wirkstofffreisetzung einer derartigen Arzneiform ist aus Abb. 1 ersichtlich.
**2.** 110 g Urapidilhydrochlorid und 55 g Mannit werden gemischt und mit einer Lösung von 10 g Kollidon®25 in einem Gemisch aus 10 g Wasser und 10 g Ethanol geknetet. Die feuchte Masse wird durch ein Sieb der lichten Maschenweite von 1,0 mm granuliert und das feuchte Granulat 40 Minuten in einem Umlufttrockenschrank bei 50°C getrocknet. Nach dem Zumischen von 1,5 g Magnesiumstearat wird das Granulat zu Kernen von 6 mm Durchmesser und 176,5 mg Gewicht verpreßt. Diese Kerne werden mit einem Filmüberzug aus Celluloseacetat mit einem Acetylgehalt von 32 %, Citroflex®2 (Weichmacher auf Basis von Citronensäureestern der Fa. Pfizer GmbH, Karlsruhe) und Titandioxid überzogen bis die Schichtdicke 70 µm beträgt. In diese Celluloseacetatschicht wird ein 0,2 mm großes Loch gebohrt.
   Die Wirkstofffreisetzung erfolgt analog Abb. 1.
**3.** Wird hingegen ein Urapidilhydrochloridkern ohne Milchzucker oder Mannit hergestellt (Zusammensetzung: 110 mg Urapadilhydrochlorid, 10 mg Kollidon®25 und 1 mg Magnesiumstearat) und wie unter Beispiel 1 mit Celluloseacetat überzogen, erhält man eine Abgabe wie Abb. 2.
   Hier erreicht man nach 15 Stunden eine Freisetzung von 83,6 %. Dies zeigt, daß auf diese Weise keine Retardform hergestellt werden kann, die sich für eine ein- oder zweimalige Verabreichung eignet.
**4.** Wird Mannit aus Beispiel 2 durch Saccharose ersetzt und der gleiche Überzug aufgebracht, erhält man eine Abgabe wie Abb. 3 (85 % Freigabe nach 15 Stunden). Hieraus ergibt sich, daß Zucker mit einer Löslichkeit von mehr als 30 Gew.-% in Wasser von 37°C nicht zur Herstellung erfindungsgemäßer Retardformen für Urapidil geeignet sind.

## Patentansprüche

1. Retardzubereitung für Urapidil nach dem Prinzip der osmotischen Pumpe, bei der sich der Wirkstoff zusammen mit Hilfsstoffen als Kern in einer mit einem Durchlaß versehenen semipermeablen Hülle befindet, dadurch gekennzeichnet, daß als Wirkstoff ein leichtlösliches Salz von Urapidil und als Hilfsstoffe Zucker und/oder Zuckeralkohole mit einer Löslichkeit von weniger als 30 Gew.-% in Wasser von 37°C verwendet werden.

2. Retardzubereitung nach Anspruch 1, dadurch gekennzeichnet, daß die Zucker und/oder Zuckeralkohole in einer Menge von mindestens 10 Gew.-% bezogen auf das Gewicht des Kerns zugegen sind.

3. Retardzubereitung nach Anspruch 1, dadurch gekennzeichnet, daß als Zucker oder Zuckeralkohol Lactose und/oder Mannit verwendet werden.

4. Retardzubereitung nach Anspruch 1, dadurch gekennzeichnet, daß 70 Gew.-% des Urapidilsalzes linear freigesetzt werden.

5. Verfahren zur Herstellung einer Retardzubereltung für Urapidil nach dem Prinzip der osmotischen Pumpe, dadurch gekennzeichnet, daß man ein leichtlösliches Salz von Urapidil zusammen mit Zuckern und/oder Zuckeralkoholen mit einer Löslichkeit von weniger als 30 Gew.-% in Wasser von 37°C und sonstigen für eine Granulierung üblichen Hilfsstoffen granuliert, zu einem Kern verpreßt und diesen mit einer semipermeablen Hülle überzieht, die mit einem Durchlaß versehen wird.

## Claims

1. Urapidil slow-release preparation on the principle of the osmotic pump, in which the active substance is located together with ancillary substances as core in a semipermeable covering provided with an opening, characterized in that a readily soluble salt of urapidil is used as active substance, and sugar and/or sugar alcohols with a solubility of less than 30% by weight in water at 37°C are used as ancillary substances.

2. Slow-release preparation according to claim 1, characterized in that the sugars and/or sugar alcohols are present in an amount of at least 10% by weight based on the weight of the core.

3. Slow-release preparation according to claim 1, characterized in that lactose and/or mannitol are used as sugar or sugar alcohol.

4. Slow-release preparation according to claim 1, characterized in that 70% by weight of the urapidil salt undergoes linear release.

5. Process for the production of a urapidil slow-release preparation on the principle of the osmotic pump, characterized in that a readily soluble salt of urapidil is granulated together with sugars and/or sugar alcohols with a solubility of less than 30% by weight in water at 37°C and other ancillary substances customary for granulation and compressed to a core, and the latter is coated with a semipermeable covering which is provided with an opening.

## Revendications

1. Préparation retard pour l'urapidil suivant le principe de la pompe osmotique, dans laquelle le principe actif se trouve en même temps que des adjuvants à titre de noyau dans une enveloppe semi-perméable pourvue d'un passage, caractérisée en ce que, à titre de principe actif, on utilise un sel facilement soluble de l'urapidil et, à titre d'adjuvant, des sucres et/ou des alcools-sucres d'une solubilité inférieure à 30% en poids dans l'eau à 37°C.

2. Préparation retard selon la revendication 1, caractérisée en ce que les sucres et/ou alcools-sucres sont présents en une proportion d'au moins 10% en poids par rapport au poids du noyau.

3. Préparation retard selon la revendication 1, caractérisée en ce que l'on utilise du lactose et/ou de la mannite à titre de sucre ou d'alcool-sucre.

4. Préparation retard selon la revendication 1, caractérisée en ce que 70% en poids du sel de l'urapidil sont libérés de manière linéaire.

5. Procédé de réalisation d'une préparation retard pour l'urapidil suivant le principe de la pompe osmotique, caractérisé en ce que l'on granule un sel facilement soluble de l'urapidil en même temps que des sucres et/ou des alcools-sucres d'une solubilité inférieure à 30% en poids dans l'eau à 37°C et certains adjuvants usuels pour la granulation, on les comprime en un noyau et on enrobe ce noyau d'une enveloppe semi-perméable, pourvue d'un passage.
